# FASCICULE DE BREVET EUROPEEN

(11) **EP 3 831 280 B1**
(45) Date de publication et mention de la délivrance du brevet: **03.09.2025**
(21) Numéro de dépôt: 20211524.2
(22) Date de dépôt: 03.12.2020
(51) Int. Cl.: A61B 5/00, A61B 5/053, A61B 5/07, A61B 5/0535

(54) **SYSTÈME POUR RÉALISER UNE MESURE D'IMPÉDANCE CARDIOGRAPHIQUE**
SYSTEM ZUR DURCHFÜHRUNG EINER KARDIOGRAPHISCHEN IMPEDANZMESSUNG
SYSTEM FOR PERFORMING AN IMPEDANCE CARDIOGRAPHY MEASUREMENT

(30) Priorité: 03.12.2019 FR 1913676
(43) Date de publication de la demande: 09.06.2021
(73) Titulaire: Sorin CRM SAS, 92140 Clamart (FR)
(72) Inventeur: MALDARI, Mirko, 75019 Paris (FR)
(74) Mandataire: Ungerer, Olaf

(56) Documents cités:
- EP-A1- 2 959 828
- US-A- 4 987 897
- US-A1- 2012 035 490
- NEAL BHATIA ET AL: "Leadless pacemakers: a contemporary review", JOURNAL OF GERIATRIC CARDIOLOGY : JGC, 1 April 2018 (2018-04-01), China, pages 249 - 253, XP055698069, Retrieved from the Internet <URL:https://www.ncbi.nlm.nih.gov/pmc/articles/PMC5997619/pdf/jgc-15-04-249.pdf> [retrieved on 20200805], DOI: 10.11909/j.issn.1671-5411.2018.04.002

## Description

La présente invention se rapporte à un système configuré pour réaliser une mesure d'impédance cardiographie.

La mesure d'impédance cardiographie peut être utilisée pour déterminer des informations physiologiques d'un patient, telle que l'activité cardiaque, comme décrit dans le document WO 0078391 A1.

Le document WO 0078391 A1 se rapporte à un dispositif médical implantable de gestion du rythme cardiaque configuré pour réaliser une pléthysmographie par impédance à partir de plusieurs sondes endocavitaires pourvues d'électrodes. Le dispositif du document WO 0078391 A1 réalise une mesure en temps réel de l'impédance dans le ventricule droit à partir de laquelle des informations hémodynamiques relatives à la performance cardiaque comme la mesure du volume systolique du ventricule gauche, la détermination de la fraction d'éjection ou le taux de remplissage peuvent être déterminées.

Toutefois, le dispositif décrit dans le document WO 0078391 A1 n'est pas adapté pour récupérer des informations respiratoires à partir de la mesure d'impédance, encore moins pour discriminer des informations respiratoires d'informations hémodynamiques. De ce fait, le dispositif décrit dans le document WO 0078391 A1 n'est pas non plus capable de différencier un artefact respiratoire dans la mesure d'impédance.

Il est cependant nécessaire de pourvoir récupérer et discriminer les informations respiratoires et hémodynamiques relatives à l'activité pulmonaire et à la circulation sanguine pour le diagnostic et le suivi d'une insuffisance cardiaque. L'insuffisance cardiaque est une incapacité du cœur à pomper une quantité de sang suffisante pour assurer un débit sanguin satisfaisant au niveau de l'ensemble du corps. L'insuffisance cardiaque a une évolution chronique et progressive, généralement lente, qui peut se compter en années.

Le document US 2019/0111268 A se rapporte à la détermination de l'impédance dans une zone extra-cardiovasculaire d'un patient au moyen d'un défibrillateur cardiaque implantable à sonde sous-cutanée. Le document US 2019/0111268 A décrit qu'un même couple d'électrodes de la sonde sous-cutanée sert à la fois de dipôle émetteur et de dipôle récepteur pour émettre et recevoir un signal d'impédance représentatif de l'impédance locale à proximité du couple d'électrodes.

Il s'avère que la détection d'événements cardiaques telle que décrite dans le document US 2019/0111268 A pour identifier un trouble du rythme cardiaque n'est pas non plus adaptée à la surveillance d'insuffisance cardiaque. En effet, la mesure d'impédance proposée dans le document US 2019/0111268 A est relative à une mesure locale, ce qui la rend peu sensible à l'activité pulmonaire et à la circulation sanguine dans les organes voisins. Or les informations respiratoires et hémodynamiques relatives à l'activité pulmonaire et à la circulation sanguine dans les organes voisins sont autant d'informations utiles au diagnostic et à la surveillance d'une insuffisance cardiaque.

Le document US 2012/0035490 A1 se rapporte à un système de plusieurs dispositifs médicaux implantables entre lesquels une mesure de transimpédance est réalisée afin de surveiller le changement de pulsatilité cardiaque.

L'objet de la présente invention est de proposer un système permettant d'améliorer et d'optimiser le diagnostic et le suivi d'une insuffisance cardiaque, notamment à partir du recueil d'informations respiratoire et hémodynamiques via une mesure d'impédance.

L'objet de la présente invention est atteint avec un système selon la revendication indépendante 1.

De manière plus générale la présente divulgation concerne un système de plusieurs dispositifs médicaux implantables pour une mesure d'impédance comprenant : un premier dispositif médical implantable comprenant au moins un dipôle émetteur formé de deux électrodes connectées à un générateur et configuré pour émettre un signal électrique, au moins un deuxième dispositif médical implantable distinct du premier dispositif médical implantable et comprenant au moins un dipôle récepteur formé de deux électrodes, le dipôle récepteur étant configuré pour recueillir le signal électrique émis au moyen du dipôle émetteur du premier dispositif médical implantable; un module d'analyse comprenant au moins un amplificateur et un détecteur d'enveloppe, l'un du premier dispositif médical implantable ou du deuxième dispositif médical implantable étant un défibrillateur automatique implantable sous-cutané ou un enregistreur en boucle sous cutanée, et l'autre du premier dispositif médical implantable ou du deuxième dispositif médical implantable étant un dispositif implantable endocavitaire.

Le fait que le système ait au moins quatre électrodes, de sorte que le dipôle émetteur soit distinct du dipôle récepteur et que l'un des dipôles soit compris dans un dispositif sous-cutanée tandis que l'autre dipôle est compris dans un dispositif endocavitaire, permet d'obtenir une mesure d'impédance plus globale et donc davantage représentative du milieu environnant, en particulier plus globale et plus représentative du milieu environnant qu'une mesure entre seulement deux électrodes d'une même sonde. En effet, le présent système permet de récupérer des informations mécaniques physiologiques au moyen des deux dispositifs distincts en analysant le signal électrique recueilli, dont l'amplitude a été modulée selon les propriétés électriques du milieu de propagation entre le dipôle émetteur et le dipôle récepteur.

Ainsi, au moyen du module d'analyse, en particulier du détecteur d'enveloppe qui est apte à effectuer une démodulation en amplitude du signal recueilli, il est possible de récupérer des informations qui peuvent être corrélées à des paramètres physiologiques tels que le débit cardiaque, la période de pré-éjection, la fraction d'éjection du ventricule gauche, la fréquence cardiaque, la fréquence respiratoire, etc., qui sont notamment utiles au diagnostic et au suivi d'une insuffisance cardiaque.

La présente divulgation peut être davantage améliorée grâce aux modes de réalisation suivants.

Selon un mode de réalisation, le module d'analyse peut en outre comprendre un convertisseur analogique-numérique et au moins un moyen de filtrage numérique configuré pour traiter le signal électrique recueilli.

Ainsi, à la suite du détecteur d'enveloppe, le signal recueilli et détecté peut être échantillonné par le convertisseur analogique-numérique du module d'analyse et filtré numériquement pour discriminer les informations respiratoires des informations hémodynamiques.

Selon un mode de réalisation, le module d'analyse peut comprendre un filtre numérique passe bas configuré pour extraire des informations respiratoires du signal électrique recueilli, en particulier un filtre numérique passe bas avec une fréquence de coupure comprise entre 0,5Hz et 5Hz, plus particulièrement avec une fréquence de coupure de 1Hz.

Ainsi, la fréquence de coupure du filtre numérique peut être ajustée en fonction des caractéristiques de chaque paramètre physiologique particulier à observer, ici des paramètres respiratoires.

Selon un mode de réalisation, le module d'analyse peut comprendre un filtre numérique passe-bande, configuré pour extraire des informations hémodynamiques du signal électrique recueilli, en particulier un filtre numérique passe bande avec une bande passante comprise entre 0,5Hz et 30Hz.

La plage de fréquences de 0,5Hz à 30Hz permet à la fois de filtrer l'artefact respiratoire en coupant les fréquences au-dessous de 0,5Hz et de filtrer les bruits à haute fréquence, c'est-à-dire les bruits dont la fréquence est au-dessus de 30Hz. Ainsi, le système peut être utilisé pour récupérer des informations hémodynamiques et respiratoires d'une même acquisition de signal au moyen de filtres numériques appropriés pour discriminer les différentes informations.

Selon un mode de réalisation, le module d'analyse peut comprendre un amplificateur passe bande à faible bruit configuré pour amplifier le signal recueilli par le dipôle récepteur.

Ainsi, le module d'analyse est configuré pour laisser passer et n'amplifier qu'une fréquence utile prédéfinie du signal recueilli.

Selon un mode de réalisation, le module d'analyse peut comprendre une pluralité d'amplificateurs à faible bruit sélectionnables selon le positionnement de chaque dipôle émetteur par rapport à chaque dipôle récepteur.

Ainsi, en fonction de l'atténuation due à la position mutuelle des dipôles, le module d'analyse du système peut sélectionner l'amplificateur adapté. Le module d'analyse du présent système est ainsi capable de s'adapter à l'anatomie de chaque patient, qui est différente pour chaque patient. En outre, de cette façon, la consommation d'énergie du système peut être optimisée.

Selon un mode de réalisation, le dispositif implantable endocavitaire peut être une capsule de stimulateur cardiaque sans sonde.

Ainsi, en utilisant le dispositif implantable sous-cutané comme émetteur, et la capsule de stimulateur cardiaque sans sonde comme récepteur et implantée dans le ventricule droit, l'information relative à la contraction de l'oreillette (« atrial kick » en anglais) peut être récupérée par la capsule de stimulateur cardiaque sans sonde étant donné que l'activité mécanique de l'oreillette modifie à la fois la quantité de sang présent dans le ventricule droit et l'orientation de la capsule de stimulateur cardiaque sans sonde. L'information relative à la contraction de l'oreillette peut être utilisée par la capsule de stimulateur cardiaque sans sonde afin d'adapter la stimulation à l'activité normale de l'oreillette.

Selon un mode de réalisation, le système peut en outre comprendre au moins une deuxième capsule de stimulateur cardiaque sans sonde pourvue d'au moins un dipôle récepteur et/ou émetteur, et le système peut être configuré pour adapter une impulsion électrique délivrée au moyen d'au moins un des dispositifs implantables du système en fonction du signal électrique recueilli par le au moins un dipôle récepteur du système.

Ainsi, lorsqu'une première capsule est implantée dans le ventricule gauche et la deuxième capsule est implantée dans le ventricule droit, le système constitue un système de resynchronisation cardiaque implantable qui est adapté au traitement de l'insuffisance cardiaque, en plus d'être configuré pour le diagnostic et le suivi d'insuffisance cardiaque.

Au moyen d'un tel système, la thérapie délivrée par application d'impulsions électriques pour traiter l'insuffisance cardiaque peut être adaptée et optimisée en tenant compte des paramètres physiologiques extraits à partir des signaux électriques recueillis par le système. Un tel système est notamment apte à synchroniser la contraction interventriculaire au moyen des capsules de stimulateur sans sonde implantées dans chaque ventricule.

Selon un mode de réalisation, le dipôle émetteur peut émettre un signal électrique à amplitude variable.

Ainsi, l'amplitude du signal électrique émis par le dipôle émetteur peut être ajustée une fois que les dispositifs implantables, et donc les dipôles émetteurs/récepteurs, sont implantés dans le corps d'un patient afin d'obtenir un rapport signal sur bruit adapté pour la détection au niveau du dipôle récepteur.

Selon un mode de réalisation, le premier dispositif médical implantable peut comprendre un module de télémétrie configuré pour communiquer avec un dispositif externe de sorte que l'amplitude du signal électrique émis par le dipôle émetteur soit ajustable par télémétrie.

Ainsi, le réglage de l'amplitude du signal électrique émis peut être davantage optimisé.

L'invention et ses avantages seront expliqués plus en détail dans ce qui suit au moyen de modes de réalisation préférés et en s'appuyant notamment sur les figures d'accompagnement suivantes, dans laquelle :
La Figure 1 représente un système selon la présente invention comprenant deux dispositifs ;
La Figure 2 représente un système selon la présente invention comprenant trois dispositifs ;
La Figure 3 représente un système selon la présente invention comprenant quatre dispositifs ;
La Figure 4 représente une vue schématisée de la propagation d'un signal électrique entre un dipôle émetteur et un dipôle récepteur du système selon la présente invention;
La Figure 5 représente schématiquement un système selon la présente invention comprenant un module d'analyse selon un premier mode de réalisation ;
La Figure 6 représente schématiquement un système selon la présente invention comprenant un module d'analyse selon un deuxième mode de réalisation ;
La Figure 7 représente schématiquement un système selon la présente invention comprenant un module d'analyse selon un troisième mode de réalisation ;
La Figure 8 représente schématiquement un système selon la présente invention comprenant un module d'analyse selon un quatrième mode de réalisation ;
La Figure 9 représente schématiquement un dispositif implantable d'un système selon la présente invention.

L'invention va maintenant être décrite plus en détail en utilisant des modes de réalisation avantageux d'une manière exemplaire et en référence aux figures. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en œuvre de la présente invention.

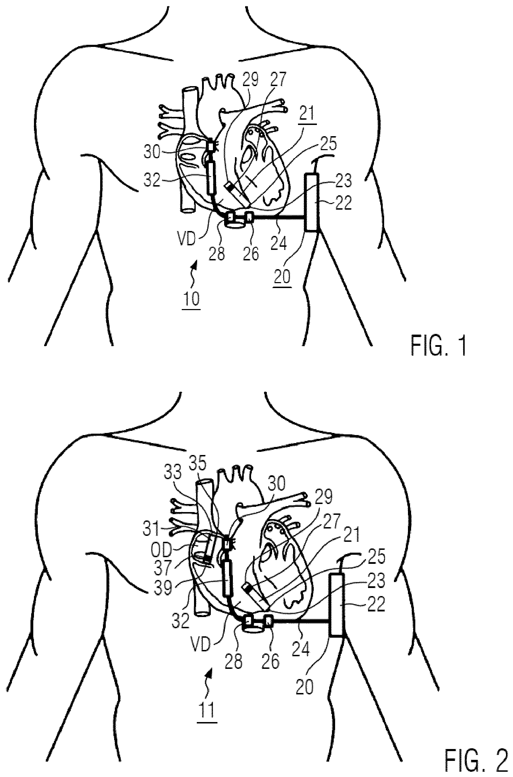

La **Figure 1** représente un système 10 selon la présente invention comprenant deux dispositifs implantables 20, 21.

Le système multi-dispositifs 10 représenté à la Figure 1 comprend un dispositif implantable sous-cutané 20 et un dispositif endocavitaire 21 qui est, dans l'exemple de la Figure 1 et dans le cadre de l'invention, une capsule de stimulateur sans sonde 21.

Dans ce qui suit, le dispositif implantable de type sous-cutané sera décrit en référence à des numéros pairs tandis que le dispositif implantable endocavitaire sera décrit en référence à des numéros impairs.

Le dispositif implantable sous-cutané 20 tel que représenté à la Figure 1 comprend un boîtier 22 et une sonde sous cutanée 24 pourvue de trois électrodes 26, 28, 30 et d'une électrode de défibrillation 32.

Le dispositif implantable sous-cutané 20 est ainsi adapté pour comprendre au moins un dipôle émetteur et un dipôle récepteur dont les électrodes de chaque dipôle sont distinctes des unes des autres. Le tableau 1 ci-dessous liste toutes les configurations de dipôles émetteurs et récepteurs qui peuvent être utilisées dans le dispositif implantable sous-cutané 20.

**[Tableau 1]**

| # | Dipôle émetteur ou récepteur |
|---|---|
| 1 | 22 - 26 |
| 2 | 22 - 28 |
| 3 | 22 - 30 |
| 4 | 22 - 32 |
| 5 | 26 - 28 |
| 6 | 26 - 30 |
| 7 | 26 - 32 |
| 8 | 28 - 30 |
| 9 | 28 - 32 |
| 10 | 30 - 32 |

Tel qu'indiqué dans le Tableau 1, l'une des électrodes peut être constituée par le boîtier 22 du dispositif implantable sous cutanée 20. Toutes les combinaisons d'électrodes peuvent être utilisées, y compris l'électrode de défibrillation 32.

Dans une variante, un enregistreur d'événement ou un enregistreur implantable en boucle comprenant au moins un couple d'électrode peut être utilisé à la place du dispositif implantable sous-cutané 10.

La capsule de stimulateur sans sonde 21 comprend une électrode de pointe 23 disposée à une extrémité distale 25 de la capsule 21, et une électrode annulaire 27 disposée vers une extrémité proximale 29 de la capsule 21. Les électrodes 23, 27 peuvent former un dipôle récepteur ou un dipôle émetteur. Notons que la présente invention ne se limite pas à l'utilisation d'une électrode de pointe et d'une électrode de type annulaire mais peut être mise en œuvre au moyen de tous types d'électrodes comprises dans une capsule de stimulateur sans sonde.

Dans une variante, un dispositif cardiaque avec une sonde endocavitaire comprenant au moins un couple d'électrodes peut être utilisé à la place de la capsule de stimulateur sans sonde 21.

Chacun du dispositif implantable sous-cutané 20 et de la capsule de stimulateur cardiaque sans sonde 21 comprend des électrodes 22, 26, 28, 30, 32 ; 23, 27 qui peuvent servir de dipôle récepteur et de dipôle émetteur. Ainsi, aussi bien le dispositif implantable sous-cutané 20 que la capsule de stimulateur cardiaque sans sonde 21 peuvent servir d'émetteur ou de récepteur dans le système 10 implantable de la présente divulgation. De plus, un praticien peut avantageusement sélectionner la configuration de dipôles émetteur et récepteur la mieux adaptée aux paramètres physiologiques qu'il souhaite recueillir. Les différentes configurations possibles de dipôle émetteur/dipôle récepteur avec le système 10 sont listées dans le Tableau 2 ci-dessous.

**[Tableau 2]**

| # | Dipôle émetteur | Dipôle récepteur |
|---|---|---|
| 1 | 22-26 | 23 - 27 |
| 2 | 22 - 28 | 23 - 27 |
| 3 | 22-30 | 23 - 27 |
| 4 | 22 - 32 | 23 - 27 |
| 5 | 26 - 28 | 23 - 27 |
| 6 | 26 - 30 | 23 - 27 |
| 7 | 26 - 32 | 23 - 27 |
| 8 | 28 - 30 | 23 - 27 |
| 9 | 28-32 | 23 - 27 |
| 10 | 30 - 32 | 23 - 27 |
| 11 | 23 - 27 | 22 - 26 |
| 12 | 23 - 27 | 22 - 28 |
| 13 | 23 - 27 | 22 - 30 |
| 14 | 23 - 27 | 22 - 32 |
| 15 | 23 - 27 | 26 - 28 |
| 16 | 23 - 27 | 26 - 30 |
| 17 | 23 - 27 | 26 - 32 |
| 18 | 23 - 27 | 28 - 30 |
| 19 | 23 - 27 | 28 - 32 |
| 20 | 23 - 27 | 30 - 32 |

Ainsi, il est possible de sélectionner la configuration des dipôles la plus sensible et/ou la plus économe en énergie, notamment au cours de la vie du patient dans laquelle les dispositifs 20, 21 sont implantés. Cette sélection peut être effectuée en temps réel au moyen d'un module de télémétrie.

L'implantation, telle qu'illustrée à la Figure 1, du dispositif implantable sous-cutané 20 et de la capsule de stimulateur cardiaque sans sonde 21, qui est implantée dans le ventricule droit VD, est adaptée pour une mesure trans-thoracique et permet de détecter les changements de volume d'une autre chambre du cœur que celle dans laquelle est implantée la capsule de stimulateur cardiaque sans sonde 21.

Par exemple, en utilisant le dispositif implantable sous-cutané 20 comme émetteur, notamment le couple d'électrodes 26, 30, et la capsule de stimulateur cardiaque sans sonde 21 implantée dans le ventricule droit VD comme récepteur (c'est-à-dire le couple d'électrodes 23, 27), l'information relative à la contraction de l'oreillette (« atrial kick » en anglais) peut être récupérée par la capsule de stimulateur cardiaque sans sonde 21 étant donné que l'activité mécanique de l'oreillette modifie à la fois la quantité de sang présent dans le ventricule droit VD et l'orientation de la capsule de stimulateur cardiaque sans sonde 21. L'information relative à la contraction de l'oreillette peut être utilisée par la capsule de stimulateur cardiaque sans sonde 21 afin d'adapter la stimulation à l'activité normale de l'oreillette.

De plus, le système 10 ayant au moins quatre électrodes, de sorte que le dipôle émetteur soit distinct du dipôle récepteur, il est possible d'obtenir une mesure d'impédance plus globale et donc davantage représentative du milieu environnant, qu'une mesure entre seulement deux électrodes d'une même sonde.

La **Figure 2** représente un système 11 selon la présente invention comprenant trois dispositifs implantables 20, 21 et 31.

Les éléments avec les mêmes références numériques déjà utilisées pour la description de la Figure 1 ne seront pas décrits à nouveau en détail, et référence est faite à leurs descriptions ci-dessus.

Le système 11 comprend un dispositif implantable de plus (31) que le système 10 décrit à la Figure 1.

Le dispositif implantable supplémentaire (31) du système 11 représenté à la Figure 2 est une capsule de stimulateur sans sonde 31, illustrée à la Figure 2, implantée dans l'oreillette droite OD. Dans une variante, la capsule de stimulateur sans sonde 31 est prévue pour être implantée dans le ventricule gauche VG. Selon la chambre dans laquelle la capsule de stimulateur sans sonde 31 est implantée, l'oreillette droite OD ou le ventricule gauche VG peut être stimulé.

De même que la première capsule de stimulateur sans sonde 21, la deuxième capsule de stimulateur sans sonde 31 comprend une électrode de pointe 33 disposée à une extrémité distale 35 de la capsule 31, et une électrode annulaire 37 disposée vers une extrémité proximale 39 de la capsule 31. Les électrodes 33, 37 peuvent former un dipôle récepteur ou un dipôle émetteur.

Notons que la présente invention ne se limite pas à l'utilisation d'une électrode de pointe et d'une électrode de type annulaire mais peut être mise en œuvre au moyen de tous types d'électrodes comprises dans une capsule de stimulateur sans sonde.

Les différentes configurations possibles de dispositifs récepteur/dispositif émetteur au moyen du système 11 comprenant trois dispositifs 20, 21, 31 sont listées dans le Tableau 3 ci-dessous. Les possibilités de configurations entre seulement deux des trois dispositifs 20, 21 31 sont également énumérées dans le Tableau 3.

**[Tableau 3]**

| # | Dispositif 20 | Capsule 21 | Capsule 31 |
|---|---|---|---|
| 1 | Dipôle émetteur | Dipôle récepteur | Dipôle récepteur |
| 2 | Dipôle récepteur | Dipôle émetteur | Dipôle récepteur |
| 3 | Dipôle récepteur | Dipôle récepteur | Dipôle émetteur |
| 4 | Dipôle émetteur | Dipôle récepteur | - |
| 5 | Dipôle émetteur | - | Dipôle récepteur |
| 6 | - | Dipôle émetteur | Dipôle récepteur |
| 7 | Dipôle récepteur | Dipôle émetteur | - |
| 8 | Dipôle récepteur | - | Dipôle émetteur |
| 9 | - | Dipôle récepteur | Dipôle émetteur |

Ainsi, le système 11 est d'autant plus adapté pour une mesure trans-thoracique et permet de détecter les changements de volume observés dans le ventricule droit VD et dans l'oreillette droite OD. En effet, le signal électrique recueilli au moyen de la capsule 21 (implantée dans le ventricule droit VD) et le signal recueilli au moyen de la capsule 31 (qui est implantée dans l'oreillette droite OD dans le mode de réalisation illustré par la Figure 2) peuvent être différents entre eux.

Tel qu'indiqué plus haut, la capsule 21 pourrait, dans une variante non nécessairement couverte par l'invention, être implantée dans le ventricule gauche VG. Quoi qu'il en soit, l'un des signaux électriques peut fournir davantage d'informations utiles que l'autre signal électrique recueilli. Le système 11 permet ainsi de déterminer le canal de propagation le mieux adapté pour la détermination des paramètres respiratoires et hémodynamiques souhaités.

Le système 11 permet en outre d'avoir aussi une vision plus exhaustive de la mesure trans-thoracique.

De plus, le système 11 est adapté pour stimuler le cœur dans l'oreillette droite OD.

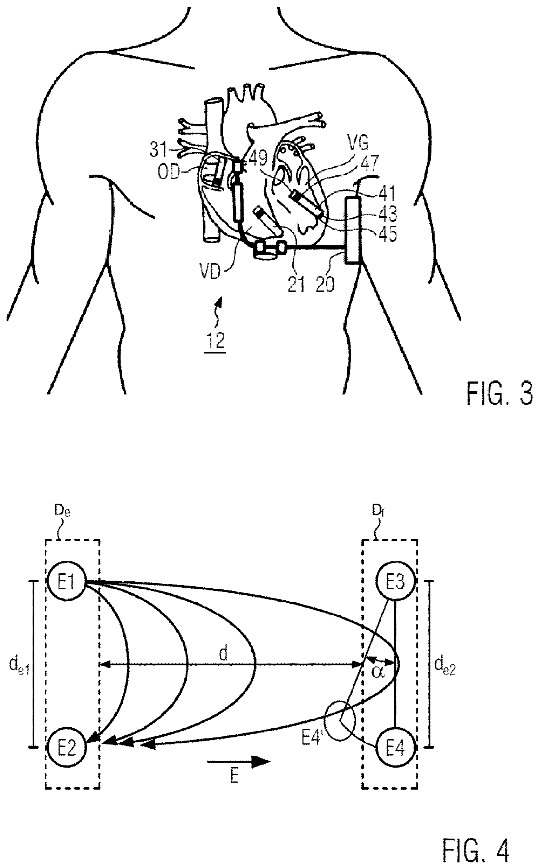

**La** **Figure 3** représente un système 12 selon la présente invention comprenant quatre dispositifs implantables 20, 21, 31 et 41.

Les éléments avec les mêmes références numériques déjà utilisées pour la description des Figures 1 et 2 ne seront pas décrits à nouveau en détail, et référence est faite à leurs descriptions ci-dessus.

Le système 12 comprend un dispositif implantable de plus (41) que le système 11 décrit à la Figure 2.

Le dispositif implantable supplémentaire (41) du système 13 représenté à la Figure 3 est une capsule de stimulateur sans sonde 41 implantée dans le ventricule gauche VG.

De même que la première capsule de stimulateur sans sonde 21 et la deuxième capsule de stimulateur sans sonde 31, la troisième capsule de stimulateur sans sonde 41 comprend une électrode de pointe 43 disposée à une extrémité distale 45 de la capsule 41, et une électrode annulaire 47 disposée vers une extrémité proximale 49 de la capsule 41. Les électrodes 43, 47 peuvent former un dipôle récepteur ou un dipôle émetteur.

Notons que la présente invention ne se limite pas à l'utilisation d'une électrode de pointe et d'une électrode de type annulaire mais peut être mise en œuvre au moyen de tous types d'électrodes comprises dans une capsule de stimulateur sans sonde.

La première capsule de stimulateur sans sonde 21 implantée dans le ventricule droit VD, la deuxième capsule de stimulateur sans sonde 31 implantée dans l'oreillette droite OD et la troisième capsule de stimulateur sans sonde 41 implantée dans le ventricule gauche VG forment un système de resynchronisation cardiaque implantable 50 dit « *leadless* » en anglais, c'est-à-dire sans sonde.

Le système de resynchronisation cardiaque implantable 50 dit « triple chambre » (VD, OD, VG) est adapté au traitement de l'insuffisance cardiaque, en plus d'être configuré pour le diagnostic et le suivi d'insuffisance cardiaque. En effet, dans le système de resynchronisation cardiaque implantable 50 la thérapie peut être optimisée en tenant compte des paramètres physiologiques recueillis à partir des signaux électriques. Le système de resynchronisation cardiaque implantable 50 est notamment apte à synchroniser la contraction intra-ventriculaire et interventriculaire au moyen de la troisième capsule de stimulateur sans sonde 41 implantée dans le ventricule gauche VG.

Aussi bien le dispositif implantable sous-cutané 20 que les capsules de stimulateur cardiaque sans sonde 21, 31, 41 peuvent servir d'émetteur ou de récepteur dans le système 12 implantable de la présente invention. De plus, un praticien peut avantageusement sélectionner la configuration de dipôles émetteur et récepteur la mieux adaptée aux paramètres physiologiques qu'il souhaite recueillir.

La **Figure 4** illustre schématiquement la propagation d'un signal électrique depuis un dipôle émetteur jusqu'à un dipôle récepteur d'un système médical implantable selon la présente invention, tel que le système 10 représenté à la Figure 1, le système 11 représenté à la Figure 2 ou le système 12 représenté à la Figure 3.

La Figure 4 illustre un dipôle émetteur Dₑ formé d'une électrode E1 et d'une électrode E2. Le dipôle émetteur Dₑ est compris dans un dispositif implantable sous cutané ou endocavitaire, tels que les dispositifs 20, 21, 31 ou 41 décrits aux Figures 1 à 3.

Par application d'un signal électrique, le dipôle émetteur Dₑ est utilisé pour créer un champ électrique E se propageant à travers des tissus d'un corps humain jusqu'à un dipôle récepteur Dᵣ. Le dipôle récepteur Dᵣ est formé d'une électrode E3 et d'une électrode E4. Le dipôle récepteur Dᵣ détecte une différence de potentiel du champ électrique E par le signal électrique détecté.

Le signal électrique détecté dépend principalement de quatre facteurs qui sont : la longueur « d » du canal de propagation, c'est-à-dire la distance entre le dipôle émetteur Dₑ et le dipôle récepteur Dᵣ; l'orientation « α » des dipôles Dₑ, Dᵣ l'un par rapport à l'autre ; les distances inter-électrodes « dₑ₁ » et « dₑ₂ » des dipôles Dₑ, Dᵣ, c'est-à-dire la distance entre les électrodes E1, E2 et la distance entre les électrodes E3, E4 ; et les propriétés électriques du milieu de propagation.

Comme le montre la Figure 4, les électrodes E3, E4 forment un dipôle récepteur dont l'orientation est différente du dipôle récepteur formé par les électrodes E3, E4'. La différence d'orientation entre les dipôles E3, E4 et E3, E4' est illustrée par l'angle α à la Figure 4.

Lorsque que le système médical implantable selon la présente invention est implanté dans un corps humain, notamment dans et au voisinage du cœur, tel qu'illustré à la Figure 1 par le système 10, à la Figure 2 par le système 11 et à la Figure 3 par le système 12, le signal électrique détecté au niveau du dipôle récepteur Dᵣ est modulé en amplitude. Cela résulte du fait que la respiration change les propriétés de l'environnement, notamment la quantité d'oxygène présente dans les poumons, ce qui fait varier l'atténuation du signal électrique au cours de sa transmission le long du canal de propagation et entraîne ainsi une variation de l'amplitude du signal électrique qui est détecté puis analysé par le système de la présente invention au moyen d'un module d'analyse.

Dans ce qui suit, le module d'analyse d'un système selon la présente invention sera davantage décrit selon plusieurs modes de réalisation.

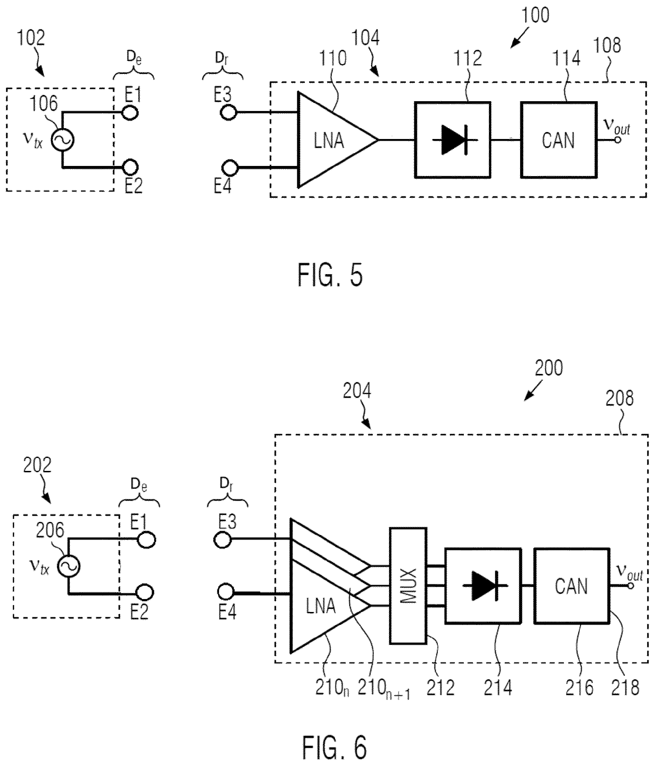

La **Figure 5** représente schématiquement un système 100 selon la présente invention comprenant un module d'analyse selon un premier mode de réalisation.

Le système 100 selon le premier mode de réalisation de l'invention comprend un premier dispositif 102 comprenant un dipôle émetteur Dₑ et un deuxième dispositif 104 comprenant un dipôle récepteur Dᵣ. Le dipôle émetteur Dₑ est formé par le couple d'électrode E1, E2 et le dipôle récepteur Dᵣ est formé par le couple d'électrode E3, E4. Le dipôle émetteur Dₑ est compris dans le dispositif implantable 102 qui est distinct de celui (104) comprenant le dipôle récepteur Dᵣ. Ainsi, les électrodes E1, E2 sont distinctes des électrodes E3, E4. De plus, un couple d'électrode est agencé de manière sous cutanée tandis que l'autre couple d'électrode est formé d'électrodes endocavitaires.

Le dipôle émetteur Dₑ est connecté à un générateur 106 à la fréquence définie f₀, tandis que le dipôle récepteur Dᵣ est connecté à un module d'analyse 108. Le générateur 106 peut être un générateur de tension ou de courant.

Notons que la fréquence f₀ doit être suffisamment élevée pour ne pas stimuler le cœur du patient en interférant avec l'activité cardiaque normale du patient.

C'est pourquoi la fréquence définie f₀ est de préférence supérieure à 1kHz, en particulier supérieure à 10kHz, de manière à ne pas interférer avec les signaux physiologiques du patient.

De manière avantageuse, le fait d'utiliser une fréquence plus basse, en particulier inférieure à 10kHz, permet de faire des économies d'énergie.

Le module d'analyse 108 comprend un amplificateur frontal à faible bruit 110 pour amplifier le signal recueilli par le dipôle récepteur Dᵣ suivi d'un détecteur d'enveloppe 112. L'amplificateur 110 peut comprendre un filtre analogique.

Le détecteur d'enveloppe 112 effectue une démodulation en amplitude du signal électrique en récupérant les informations des dipôles qui peuvent être corrélées aux paramètres hémodynamiques et à la fréquence respiratoire.

Le détecteur d'enveloppe 112 est suivi d'un convertisseur analogique-numérique 114 configuré pour échantillonner le signal électrique recueilli.

La **Figure 6** représente schématiquement un système 200 selon la présente invention comprenant un module d'analyse selon un deuxième mode de réalisation.

Le système 200 selon le deuxième mode de réalisation de l'invention comprend un premier dispositif 202 comprenant un dipôle émetteur Dₑ et un deuxième dispositif 204 comprenant un dipôle récepteur Dᵣ.

De même que dans le premier mode de réalisation, le dipôle émetteur Dₑ est formé par le couple d'électrodes E1, E2 et le dipôle récepteur Dᵣ est formé par le couple d'électrodes E3, E4. Le dipôle émetteur Dₑ est compris dans le dispositif implantable 202 qui est distinct de celui (204) comprenant le dipôle récepteur Dᵣ. Ainsi, les électrodes E1, E2 sont distinctes des électrodes E3, E4. De plus, un couple d'électrodes est agencé de manière sous cutanée tandis que l'autre couple d'électrodes est formé d'électrodes endocavitaires.

Le dipôle émetteur Dₑ est connecté à un générateur 206 à la fréquence définie f₀, tandis que le dipôle récepteur Dᵣ est connecté à un module d'analyse 208.

Le dipôle émetteur Dₑ du système 200 peut émettre un signal électrique à amplitude variable. Ainsi, l'amplitude du signal électrique émis par le dipôle émetteur Dₑ peut être ajustée une fois que les dispositifs implantables 202, 204, et donc les dipôles émetteurs/récepteurs, sont implantés dans le corps d'un patient afin d'obtenir un rapport signal sur bruit adapté pour la détection au niveau du dipôle récepteur Dᵣ.

Le dispositif implantable 204 peut comprendre un module de télémétrie (non visible sur la Figure 6) connecté au module d'analyse 208 et configuré pour communiquer des données à un dispositif externe (non visible sur la Figure 6) de sorte que, selon le signal électrique recueilli, l'amplitude du signal électrique émis par le dipôle émetteur Dₑ soit ajustable par télémétrie.

Selon le deuxième mode de réalisation de l'invention, le module d'analyse 208 comprend une pluralité de n amplificateurs à faible bruit 210ₙ sélectionnables selon le positionnement du dipôle émetteur Dₑ par rapport au dipôle récepteur Dᵣ. En effet, le positionnement relatif du dipôle émetteur Dₑ par rapport à chaque dipôle récepteur Dᵣ a une influence sur les propriétés du canal de propagation du signal électrique. Ainsi, en fonction de l'atténuation du canal due à la position mutuelle des dispositifs implantables 202, 204, le dispositif implantable 204 comprenant le dipôle récepteur Dᵣ peut sélectionner l'amplificateur à faible bruit dont le gain est le mieux adapté au signal recueilli. De cette façon, la consommation énergétique du système 200 peut être optimisée en activant seulement l'amplificateur à faible bruit nécessaire pour avoir une détection du signal électrique suffisante pour la mesure, c'est-à-dire qui respecte un certain rapport signal sur bruit prédéfini.

Dans le module d'analyse 208 du dispositif implantable 204, la pluralité d'amplificateurs à faible bruit 210ₙ sélectionnables est suivi d'un multiplexeur 212 lui-même suivi d'un détecteur d'enveloppe 214.

Le module d'analyse 208 comprend en outre un convertisseur analogique-numérique 216 et des filtres numériques 218 qui sont configurés pour traiter le signal électrique recueilli au moyen du dipôle récepteur Dᵣ. Ainsi, à la suite du détecteur d'enveloppe 214, le signal recueilli et détecté peut être échantillonné par le convertisseur analogique-numérique 216 du module d'analyse 208 et filtré numériquement pour discriminer les informations respiratoires des informations hémodynamiques, tel qu'expliqué dans ce qui suit.

Le module d'analyse 208 comprend un moyen de filtrage numérique. Plus particulièrement, le module d'analyse 208 comprend un filtre numérique passe bas configuré pour extraire des informations respiratoires du signal électrique recueilli, en particulier un filtre numérique passe bas avec une fréquence de coupure f_{c} comprise entre 0,5 et 5Hz, en particulier f_{c}=1 Hz.

Le module d'analyse 208 comprend en outre un filtre numérique passe-bande, configuré pour extraire des informations hémodynamiques du signal électrique recueilli, en particulier un filtre numérique passe bande avec une bande passante comprise entre 0,5Hz et 30Hz, en particulier de 1Hz à 10Hz.

La plage de fréquence de 0,5Hz à 30Hz permet à la fois de filtrer l'artefact respiratoire en coupant les fréquences au-dessous de 0,5Hz et de filtrer les bruits à haut fréquence, c'est-à-dire les bruits dont la fréquence est au-dessus de 30Hz, notamment les bruits à haute fréquence dont la fréquence est de l'ordre de 50 à 60Hz.

Notons que la sélection de la plage de fréquence pour le filtre numérique passe bande peut permettre de faire des économies en terme de traitement numérique.

De ce fait, le système 200 peut être utilisé pour récupérer des informations hémodynamiques et respiratoires d'une même acquisition de signal au moyen de filtres numériques 218 appropriés pour discriminer les différentes informations.

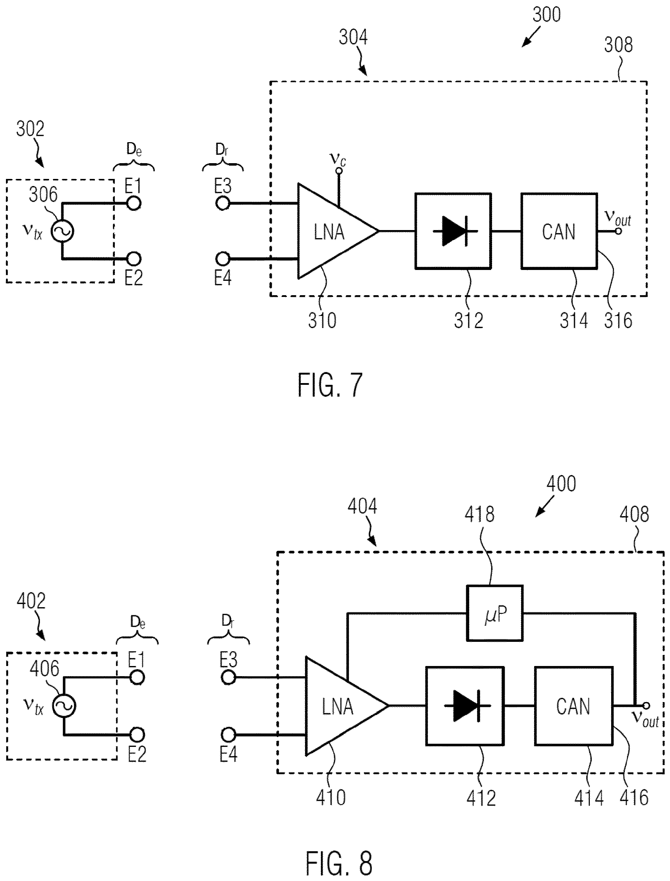

La **Figure 7** représente schématiquement un système 300 selon la présente invention comprenant un module d'analyse selon un troisième mode de réalisation.

Le système 300 selon le troisième mode de réalisation de l'invention comprend un premier dispositif 302 comprenant un dipôle émetteur Dₑ et un deuxième dispositif 304 comprenant un dipôle récepteur Dᵣ.

De même que dans les premier et deuxième modes de réalisation, le dipôle émetteur Dₑ est formé par le couple d'électrodes E1, E2 et le dipôle récepteur Dᵣ est formé par le couple d'électrodes E3, E4. Le dipôle émetteur Dₑ est compris dans le dispositif implantable 302 qui est distinct de celui (304) comprenant le dipôle récepteur Dᵣ. Ainsi, les électrodes E1, E2 sont distinctes des électrodes E3, E4. De plus, un couple d'électrodes est agencé de manière sous cutanée tandis que l'autre couple d'électrodes est formé d'électrodes endocavitaires.

Le dipôle émetteur Dₑ est connecté à un générateur 306 à la fréquence définie f₀, tandis que le dipôle récepteur Dᵣ est connecté à un module d'analyse 308.

Le module d'analyse 308 comprend un amplificateur à gain variable 310 suivi d'un détecteur d'enveloppe 312. Le gain de l'amplificateur à gain variable 310 est contrôlé en ajustant une tension de commande Vc.

De la même manière que le module d'analyse 208 décrit en référence à la Figure 6, le module d'analyse 308 comprend un convertisseur analogique-numérique 314 et des filtres numériques 316. Le convertisseur analogique-numérique 314 et les filtres numériques 316 sont identiques à ceux du module d'analyse 208 de la Figure 6. De ce fait, pour le convertisseur analogique-numérique et les filtres numériques déjà utilisés pour la description de la Figure 7, référence est faite à leurs descriptions ci-dessus.

La **Figure 8** représente schématiquement un système 400 selon la présente invention comprenant un module d'analyse selon un quatrième mode de réalisation.

Le système 400 selon le quatrième mode de réalisation de l'invention comprend un premier dispositif 402 comprenant un dipôle émetteur Dₑ et un deuxième dispositif 404 comprenant un dipôle récepteur Dᵣ.

De même que dans les modes de réalisation précédents, le dipôle émetteur Dₑ est formé par le couple d'électrodes E1, E2 et le dipôle récepteur Dᵣ est formé par le couple d'électrodes E3, E4. Le dipôle émetteur Dₑ est compris dans le dispositif implantable 402 qui est distinct de celui (404) comprenant le dipôle récepteur Dᵣ. Ainsi, les électrodes E1, E2 sont distinctes des électrodes E3, E4. De plus, un couple d'électrodes est agencé de manière sous cutanée tandis que l'autre couple d'électrodes est formé d'électrodes endocavitaires ou épicardiques.

Le dipôle émetteur Dₑ est connecté à un générateur 406 à la fréquence définie f₀, tandis que le dipôle récepteur Dᵣ est connecté à un module d'analyse 408.

Le module d'analyse 408 est compris dans le dispositif implantable 404 comprenant le dipôle récepteur Dᵣ.

Le module d'analyse 408 comprend un amplificateur à gain programmable 410 suivi d'un détecteur d'enveloppe 412, d'un convertisseur analogique-numérique 414 et de filtres numériques 416.

Le convertisseur analogique-numérique 414 et de filtres numériques 416 sont identiques à ceux du module d'analyse 308 de la Figure 7. De ce fait, pour le convertisseur analogique-numérique et les filtres numériques déjà utilisés pour la description de la Figure 7, référence est faite à leurs descriptions ci-dessus.

Le gain de l'amplificateur à gain programmable 410 est contrôlé numériquement au moyen d'un microcontrôleur interne 418.

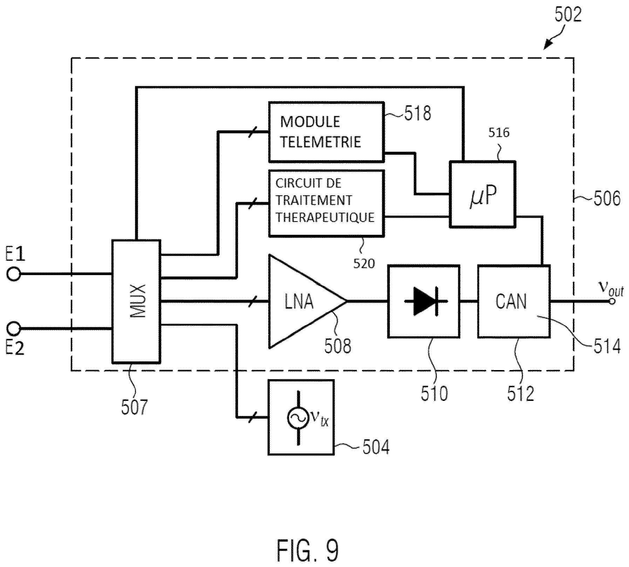

La **Figure 9** représente schématiquement un dispositif implantable 502 compris dans un système selon la présente invention.

Le dispositif implantable 502 est un défibrillateur automatique implantable sous-cutané.

Dans une variante non nécessairement couverte par l'invention, le dispositif implantable 502 est un enregistreur en boucle sous cutané. Dans une autre variante, le dispositif implantable 502 est un dispositif implantable endocavitaire. Le dispositif implantable 502 comprend deux électrodes E1, E2 qui peuvent aussi bien former un dipôle récepteur qu'un dipôle émetteur.

Le dispositif implantable 502 comprend un générateur 504 qui peut servir de générateur au dipôle émetteur E1, E2 - lorsque le couple E1, E2 forme un dipôle émetteur.

Le dispositif implantable 502 comprend un module d'analyse et de contrôle 506.

Dans le module d'analyse et de contrôle 506, à la suite d'un multiplexeur 507, le dispositif implantable 502 comprend un amplificateur à faible bruit 508, un détecteur d'enveloppe 510, un convertisseur analogique-numérique 512 et des filtres numériques 514.

Tel qu'illustré à la Figure 9, le module d'analyse et de contrôle 506 comprend en outre un microprocesseur interne 516 connecté au convertisseur analogique-numérique 512 et aux filtres numériques 514 ainsi qu'à un module de télémétrie 518 et à un circuit de traitement thérapeutique 520.

Le système selon la présente invention est configuré pour récupérer des informations hémodynamiques et respiratoires d'une même acquisition de signal, notamment au moyen des filtres numériques appropriés pour discriminer les différentes informations.

Notons que dans chaque mode de réalisation, chaque dipôle d'électrodes peut aussi bien servir de dipôle émetteur que de dipôle récepteur. Le système selon l'invention permet de sélectionner la configuration de dipôle la plus sensible à la détection et/ou la plus économe en énergie.

Les modes de réalisation décrits sont simplement des configurations possibles et il faut garder à l'esprit que les caractéristiques individuelles des différents modes de réalisation peuvent être combinées entre elles ou fournies indépendamment les unes des autres. La référence au singulier doit également être interprétée comme se rapportant au pluriel. L'invention est définie par les revendications jointes.

## Revendications

1. Système de plusieurs dispositifs médicaux implantables pour une mesure d'impédance comprenant :
un premier dispositif médical implantable comprenant au moins un dipôle émetteur (Dₑ) formé de deux électrodes (F1, E2) connectées à un générateur (16) et configuré pour émettre un signal électrique ;
au moins un deuxième dispositif médical implantable distinct du premier dispositif médical implantable et comprenant au moins un dipôle récepteur (Dᵣ) formé de deux électrodes (E3, E4), le dipôle récepteur (14) étant configuré pour recueillir le signal électrique émis au moyen du dipôle émetteur (Dₑ) du premier dispositif médical implantable ; et
un module d'analyse (108, 208, 308, 408, 506) connecté au dipôle récepteur (Dᵣ) du deuxième dispositif médical implantable et comprenant au moins un amplificateur (110, 210, 310, 410, 508) suivi d'un détecteur d'enveloppe (112, 214, 312, 421, 510), dans lequel l'amplificateur (110, 210, 310, 410, 508) est configuré pour amplifier le signal électrique recueilli par le dipôle récepteur (Dᵣ) et le détecteur d'enveloppe (112, 214, 312, 421, 510) est configuré pour effectuer une démodulation en amplitude du signal électrique recueilli en récupérant des informations respiratoires et hémodynamiques du signal électrique recueilli à des fins d'utilisation par le deuxième dispositif médical implantable, le premier dispositif médical implantable étant un défibrillateur automatique implantable sous-cutané (20), et le deuxième dispositif médical implantable étant une capsule de stimulateur cardiaque sans sonde (21, 31, 41) destinée à être implantée dans le ventricule droit (VD).

2. Le système selon la revendication 1, dans lequel le module d'analyse (108, 208, 308, 408, 506) comprend en outre un convertisseur analogique-numérique (114, 216, 314, 414, 512) et au moins un moyen de filtrage numérique configuré pour traiter le signal électrique recueilli.

3. Le système selon la revendication 2, dans lequel le module d'analyse (108, 208, 308, 408, 506) comprend un filtre numérique passe-bas configuré pour extraire les informations respiratoires du signal électrique recueilli, en particulier un filtre numérique passe-bas avec une fréquence de coupure comprise entre 0,5Hz et 5Hz, plus en particulier avec une fréquence de coupure de 1Hz.

4. Le système selon l'une des revendications 2 à 3, dans lequel le module d'analyse (108, 208, 308, 408, 506) comprend un filtre numérique passe-bande, configuré pour extraire les informations hémodynamiques du signal électrique recueilli, en particulier un filtre numérique passe-bande avec une bande passante comprise entre 0,5Hz et 30Hz.

5. Le système selon l'une des revendications précédentes, dans lequel le module d'analyse (108, 208, 308, 408, 506) comprend un amplificateur passe-bande à faible bruit configuré pour amplifier le signal recueilli par le dipôle récepteur (Dᵣ).

6. Le système selon l'une des revendications précédentes, dans lequel le module d'analyse (108, 208, 308, 408, 506) comprend une pluralité d'amplificateurs (210ₙ) à faible bruit sélectionnables selon le positionnement de chaque dipôle émetteur (Dₑ) par rapport à chaque dipôle récepteur (Dᵣ).

7. Le système selon la revendication 1, comprenant en outre au moins une deuxième capsule de stimulateur cardiaque sans sonde (21, 31, 41) pourvue d'au moins un dipôle récepteur (Dᵣ), et
le système étant configuré pour adapter une impulsion électrique délivrée au moyen d'au moins un des dispositifs implantables (20, 21, 31, 41) du système en fonction du signal électrique recueilli par au moins un dipôle récepteur (Dᵣ) du système.

8. Le système selon l'une des revendications précédentes, dans lequel le dipôle émetteur (Dₑ) émet un signal électrique à amplitude variable.

9. Le système selon la revendication 8, dont le premier dispositif médical implantable comprend un module de télémétrie (518) configuré pour communiquer avec un dispositif externe de sorte que l'amplitude du signal électrique émis par le dipôle émetteur (Dₑ) est ajustable par télémétrie.

## Patentansprüche

1. Mehrkomponentensystem medizinischer implantierbarer Vorrichtungen zur Impedanzmessung, umfassend:
eine erste implantierbare medizinische Vorrichtung, die mindestens ein Sendedipol (De) umfasst, gebildet aus zwei Elektroden (E1, E2), die mit einem Generator (16) verbunden sind und dazu eingerichtet sind, ein elektrisches Signal zu senden;
mindestens eine zweite implantierbare medizinische Vorrichtung, die von der ersten implantierbaren medizinischen Vorrichtung getrennt ist und mindestens ein Empfangsdipol (Dr) umfasst, gebildet aus zwei Elektroden (E3, E4), wobei der Empfangsdipol (14) dazu eingerichtet ist, das mittels des Sendedipols (De) der ersten implantierbaren medizinischen Vorrichtung gesendete elektrische Signal zu empfangen; und
ein Analysemodul (108, 208, 308, 408, 506), das mit dem Empfangsdipol (Dr) der zweiten implantierbaren medizinischen Vorrichtung verbunden ist und mindestens einen Verstärker (110, 210, 310, 410, 508) umfasst, gefolgt von einem Hüllkurvendetektor (112, 214, 312, 421, 510), wobei der Verstärker (110, 210, 310, 410, 508) dazu eingerichtet ist, das vom Empfangsdipol (Dr) empfangene elektrische Signal zu verstärken, und der Hüllkurvendetektor (112, 214, 312, 421, 510) dazu eingerichtet ist, eine Amplitudendemodulation des empfangenen elektrischen Signals durchzuführen, um respiratorische und hämodynamische Informationen aus dem empfangenen elektrischen Signal zu extrahieren, zur Verwendung durch die zweite implantierbare medizinische Vorrichtung;
wobei die erste implantierbare medizinische Vorrichtung ein subkutan implantierbarer automatischer Defibrillator (20) ist und die zweite implantierbare medizinische Vorrichtung eine kabellose Herzschrittmacherkapsel (21, 31, 41) ist, die zur Implantation in den rechten Ventrikel (VD) vorgesehen ist.

2. Das System nach Anspruch 1, wobei das Analysemodul (108, 208, 308, 408, 506) ferner einen Analog-Digital-Wandler (114, 216, 314, 414, 512) und mindestens ein digitales Filtermittel umfasst, das dazu eingerichtet ist, das empfangene elektrische Signal zu verarbeiten.

3. Das System nach Anspruch 2, wobei das Analysemodul (108, 208, 308, 408, 506) ein digitales Tiefpassfilter umfasst, das dazu eingerichtet ist, respiratorische Informationen aus dem empfangenen elektrischen Signal zu extrahieren, insbesondere ein digitales Tiefpassfilter mit einer Grenzfrequenz zwischen 0,5 Hz und 5 Hz, insbesondere mit einer Grenzfrequenz von 1 Hz.

4. Das System nach einem der Ansprüche 2 bis 3, wobei das Analysemodul (108, 208, 308, 408, 506) ein digitales Bandpassfilter umfasst, das dazu eingerichtet ist, hämodynamische Informationen aus dem empfangenen elektrischen Signal zu extrahieren, insbesondere ein digitales Bandpassfilter mit einer Bandbreite zwischen 0,5 Hz und 30 Hz.

5. Das System nach einem der vorhergehenden Ansprüche, wobei das Analysemodul (108, 208, 308, 408, 506) einen rauscharmen Bandpassverstärker umfasst, der dazu eingerichtet ist, das vom Empfangsdipol (Dr) empfangene Signal zu verstärken.

6. Das System nach einem der vorhergehenden Ansprüche, wobei das Analysemodul (108, 208, 308, 408, 506) eine Vielzahl von rauscharmen Verstärkern (210n) umfasst, die je nach Positionierung jedes Sendedipols (De) relativ zu jedem Empfangsdipol (Dr) auswählbar sind.

7. Das System nach Anspruch 1, ferner umfassend mindestens eine zweite kabellose Herzschrittmacherkapsel (21, 31, 41), die mit mindestens einem Empfangsdipol (Dr) versehen ist, wobei das System dazu eingerichtet ist, einen elektrischen Impuls, der mittels mindestens einer der implantierbaren Vorrichtungen (20, 21, 31, 41) des Systems abgegeben wird, basierend auf dem durch mindestens einen Empfangsdipol (Dr) des Systems empfangenen elektrischen Signal anzupassen.

8. Das System nach einem der vorhergehenden Ansprüche, wobei der Sendedipol (De) ein elektrisches Signal mit variabler Amplitude sendet.

9. Das System nach Anspruch 8, wobei die erste implantierbare medizinische Vorrichtung ein Telemetriemodul (518) umfasst, das dazu eingerichtet ist, mit einem externen Gerät zu kommunizieren, sodass die Amplitude des vom Sendedipol (De) gesendeten elektrischen Signals per Telemetrie einstellbar ist.

## Claims

1. A system of multiple implantable medical devices for measuring impedance, the system comprising:
a first implantable medical device comprising at least one transmitting dipole (Dₑ) formed by two electrodes (E1, E2) connected to a generator (16) and configured to transmit an electrical signal;
at least one second implantable medical device distinct from the first implantable medical device and comprising at least one receiving dipole (Dᵣ) formed of two electrodes (E3, E4), the receiving dipole (14) being configured to collect the electrical signal transmitted by means of the transmitting dipole (Dₑ) of the first implantable medical device; and
an analysis module (108, 208, 308, 408, 506) connected to the receiving dipole (Dᵣ) of the second implantable medical device and comprising at least one amplifier (110, 210, 310, 410, 508) followed by an envelope detector (112, 214, 312, 421, 510), wherein the amplifier (110, 210, 310, 410, 508) is configured to amplify the electrical signal collected by the receiving dipole (Dᵣ), and wherein the envelope detector (112, 214, 312, 421, 510) is configured to demodulate an amplitude of the collected electrical signal by retrieving respiratory and hemodynamic information from the collected electrical signal for use by the second implantable medical device,
the first implantable medical device being a subcutaneous implantable cardioverter defibrillator (20), and the second implantable medical device being a leadless cardiac pacemaker capsule (21, 31, 41) implanted in the right ventricle (RV).

2. The system of claim 1, wherein the analysis module (108, 208, 308, 408, 506) further comprises an analog-to-digital converter (114, 216, 314, 414, 512) and at least one digital filtering means configured to process the collected electrical signal.

3. The system of claim 2, wherein the analysis module (108, 208, 308, 408, 506) comprises a low-pass digital filter configured to extract the respiratory information from the collected electrical signal, in particular a low-pass digital filter with a cutoff frequency between 0.5Hz and 5Hz, more particularly with a cutoff frequency of 1Hz.

4. The system of any one of claims 2 to 3, wherein the analysis module (108, 208, 308, 408, 506) comprises a band-pass digital filter configured to extract the hemodynamic information from the collected electrical signal, in particular a band-pass digital filter with a bandwidth between 0.5Hz and 30Hz.

5. The system of any one of the preceding claims, wherein the analysis module (108, 208, 308, 408, 506) comprises a low-noise band-pass amplifier configured to amplify the signal collected by the receiving dipole (Dᵣ).

6. The system of any one of the preceding claims, wherein the analysis module (108, 208, 308, 408, 506) comprises a plurality of low-noise amplifiers (210ₙ) being selectable based on a positioning of each transmitting dipole (Dₑ) relative to each receiving dipole (Dᵣ).

7. The system of claim 1, further comprising at least one second leadless cardiac pacemaker capsule (21, 31, 41) provided with at least one receiving dipole (Dᵣ), and
the system being configured to adapt an electrical pulse, provided by means of at least one of the implantable devices (20, 21, 31, 41) of the system, based on the electrical signal collected by at least one receiving dipole (Dᵣ) of the system.

8. The system of any one of the preceding claims, wherein the transmitting dipole (Dₑ) transmits an electrical signal having a variable amplitude.

9. The system of claim 8, wherein the first implantable medical device comprises a telemetry module (518) configured to communicate with an external device such that the amplitude of the electrical signal transmitted by the transmitting dipole (Dₑ) is adjustable via telemetry.
